# EUROPEAN PATENT APPLICATION

(11) **EP 4 353 815 A1**
(43) Date of publication of application: **17.04.2024**
(21) Application number: 22816499.2
(22) Date of filing: 03.06.2022
(51) Int. Cl.: C12N 5/00, C12N 5/078, C12N 5/071, B01D 21/00, B01D 35/02, B01D 15/38, B01D 61/14

(54) **METHOD FOR ISOLATING EXTRACELLULAR VESICLE USING SALT FRACTIONAL PRECIPITATION**

(30) Priority: 04.06.2021 KR 20210072868
(71) Applicant: Institute for Basic Science, Yuseong-gu Daejeon 34126 (KR); Ulsan National Institute of Science and Technology (UNIST), Eonyang-eup Ulju-gun Ulsan 44919 (KR)
(72) Inventor: CHO, Yoon-Kyoung, Ulju-gun, Ulsan 44920 (KR); SUNKARA, Vijaya, Ulju-gun, Ulsan 44919 (KR)
(74) Representative: Mewburn Ellis LLP
(86) International application number: PCT/KR2022/007932
(87) International publication number: WO 2022/255840

(57) **Abstract**

The present invention relates to a method and kit for isolating an extracellular vesicle (EV) from a biological sample using fractional precipitation. According to the method and kit, rapid isolation is possible, and an EV can be effectively isolated from various types of biological samples regardless of the sample sources. Unlike a method for isolating an EV on the basis of an existing precipitation method, the present invention can easily remove a salt by washing, and, thus, has the benefit of being able to isolate a highly pure EV with a high yield rate and can be applied to both small-scale EV isolation in research and diagnostic fields and large-scale EV isolation for industrial applications.

## Description

### Technical Field

The present invention relates to a method of isolating extracellular vesicles (EVs) from a biological sample, and more specifically, to a method for isolating extracellular vesicles comprising a step of adding a salt to a biological sample, and a kit for isolation of extracellular vesicles.

### Background Art

Extracellular vesicles (EVs) are fine particles surrounded by a lipid bilayer, which are capable of transporting molecules, including proteins, lipids, and nucleic acids, and are released by almost all types of cells, both eukaryotic and prokaryotic. They participate in a variety of biophysical processes, including intercellular communication and signal transduction. Initially, they were known as cellular dust released during cell death or apoptosis. However, recent studies have reported that they are important messengers in intercellular signaling and are associated with cancer cell metastasis, immune function, tissue regeneration, and the like. In addition, they are also known to serve as biomarkers related to the diagnosis of certain diseases such as cancer. Extracellular vesicles can be divided into exosomes, which are tens of nanometers in size, and microvesicles, which are hundreds of nanometers in size. Since the isolation of extracellular vesicles from complex biological samples can be used for various purposes in medicine, such as disease diagnosis, treatment monitoring, and targeted therapy through gene or drug delivery, technology for efficiently isolating these extracellular vesicles from biological fluids is required.

There are several methods for isolating extracellular vesicles. The most widely used method is ultracentrifugation (UC), but this method requires expensive equipment and long processing time and has low yield. Therefore, other isolation methods and kits based on the physicochemical properties of extracellular vesicles have been developed. For example, several reagents based on polymers (polyethylene glycol, volume-excluding polymers such as polyethylene glycol) have been developed and commercialized for the isolation of extracellular vesicles from various biological samples (US 9,005,888 B, US 9,671,321 B, and US 2020/0179827 A). However, there is a disadvantage that the polymer reagent remaining in the extracellular vesicle sample after isolation may interfere with various analytical reactions that are performed in subsequent steps.

Additionally, affinity-based isolation methods based on the surface biochemical properties of extracellular vesicles have also been developed (heparin: US 9,829,483 B; Balaj et al., Sci Rep. 2015; 5: 10266; SiC: US 2019/0078078 A; Tim4: Nakai et al., Sci Rep. 2016; 6: 33935; mag capture; Qiagen; Tim4, Nakai et al., Sci Rep. 2016; mag capture, Ghosh et al., PLoS One, 2014). These methods have limitations, such as the fact that molecules with surface affinity properties are isolated together with extracellular vesicles, the fact that certain antibodies specific to extracellular vesicles are needed, and the fact that the isolation yield of extracellular vesicles is not reproducible.

As a method of isolating extracellular vesicles using a salt, an attempt has been made to precipitate extracellular vesicles by charge neutralization of phosphatidylserine on the EV surface using 0.1M sodium acetate buffer (pH 4.75) (J Immunol Methods. 2014, 407, 120-6, US 9,835,626 B). In this study, a higher yield of extracellular vesicles was obtained at low pH, and this pH dependence is actually not ideal for the process of isolating extracellular vesicles from neutral biological samples. This was also found in a study comparing the isolation efficiency of extracellular vesicles from plasma and urine samples (Ana Gamez-Valero et al., Front. Immunol., 2015; 6: 6), and the precipitation method using sodium acetate had a significantly lower recovery rate of extracellular vesicles compared to other test methods. Therefore, there is a great need to develop a better method for efficiently isolating extracellular vesicles from biological samples.

Accordingly, the present inventors have made extensive efforts to develop a quick, efficient and user-friendly method that may be used to isolate EVs from biological samples regardless of sample volume, type, or sample source, is scalable, and is capable of isolating EVs in high yield. As a result, the present inventors have found that an extracellular vesicle isolation method using a salt such as ammonium sulfate (AS) is a user-friendly extracellular vesicle isolation method that is capable of isolating extracellular vesicles quickly and in high yield from various biological samples such as plasma, serum, urine, culture media, etc., regardless of sample volume, type, source, etc., and, in particular have found that, when a multi-step salt precipitation method based on the addition of gradually increasing concentrations of salt is used, it is capable of removing various protein impurities with high efficiency by precipitating the impurities, thereby completing the present invention.

The information disclosed in this Background section is provided only for enhancement of understanding of the background of the present invention, and therefore it may not include information that forms the prior art that is already obvious to those skilled in the art.

### Summary of the Invention

An object of the present invention is to provide a method of effectively isolating extracellular vesicles (EVs) from biological samples such as biological fluid samples or biological tissue samples.

Another object of the present invention is to provide a kit for isolating extracellular vesicles (EVs).

To achieve the above objects, the present invention provides a method of isolating extracellular vesicles (EVs) from a biological fluid sample, the method comprising: (a) adding a salt to the biological fluid sample to a concentration ranging from 0.1 M to a saturation concentration; (b) separating an aggregated or precipitated extracellular vesicle fraction and a supernatant from the sample to which the salt has been added; and (c) isolating extracellular vesicles by desalting the extracellular vesicle fraction separated in step (b).

The present invention also provides a method of isolating extracellular vesicles (EVs) from a biological tissue sample, the method comprising: (a) lysing or grinding and clarifying the biological tissue sample; (b) adding a salt to the clarified sample to a concentration ranging from 0.1 M to a saturation concentration; (c) separating an aggregated or precipitated extracellular vesicle fraction and a supernatant from the sample to which the salt has been added; and (d) isolating extracellular vesicles by desalting the extracellular vesicle fraction separated in step (c).

The present invention also provides a kit for isolating extracellular vesicles (EVs) comprising a salt and a buffer.

### Brief Description of Drawings

FIG. 1 is a schematic view showing multi-step salt fractional precipitation (SP) for isolating extracellular vesicles (EVs) from a biological fluid.
FIG. 2 is a schematic view showing EV isolation from plasma by six-step SP.
FIG. 3 depicts graphs showing the results of analysis of the total protein contents and EV contents of fractions isolated from plasma by six-step SP. FIG. 3A shows a comparison of the total protein contents (BCA assay); FIG. 3B shows a comparison of EV recovery rate; and FIG. 3C shows the results of ELISA for EV markers, CD9-CD81 (membrane surface) and Alix (inner). It can be seen that the EV content is highest in F3 and F4 fractions.
FIG. 4 depicts graphs showing the results of ELISA for albumin and lipoproteins, which are protein impurities present in the fractions separated from plasma by six-step SP. FIG. 4A shows albumin; FIG. 4B shows Apo-AI; FIG. 4C shows Apo-B; and FIG. 4D shows Apo-E. It can be seen that the largest amounts of impurity proteins are present in F5 and F6 fractions.
FIG. 5 depicts graphs showing comparative ELISA results for EV markers (CD9-CD81 (membrane surface) and Alix (inner)) in fractions formed by three-step SP, and shows the results of comparison using solid AS (FIG. 5A) and liquid AS (FIG. 5B) at the same concentration.
FIG. 6 depicts graphs showing the results of ELISA for EV markers (CD9-CD81 and Alix) in fractions formed by three-step SP using 0.4 mL plasma (FIG. 6A) and 4 mL plasma (FIG. 6B). It can be seen that SP is scalable and may be used for both small and large samples.
FIG. 7 shows the characterization of EVs isolated by two-step SP from fresh or freeze-thawed plasma or serum. FIG. 7A shows the results of total protein quantification (BCA); FIG. 7B shows EV recovery rate; FIG. 7C shows the results of ELISA for EV markers; and FIG. 7D shows the results of NTA analysis.
FIG. 8 shows the results of analyzing the yields and total protein contents of EVs isolated from plasma by two-step SP and EVs isolated using other isolation kits such as ExoQuick and exoEasy. Specifically, FIG. 8 depicts graphs showing the results of analyzing EVs by BCA for total protein quantification (FIG. 8A) and the percent recovery of EVs (FIG. 8B). It is shown that EVs isolated by SP has a higher EV yield and lower protein impurity content than EVs isolated by other methods.
FIG. 9 depicts graphs showing the results of NTA analysis of EVs isolated from plasma by two-step SP and EVs isolated using other isolation kits such as ExoQuick and exoEasy, and shows the concentration (FIG. 9A) and size distribution (FIG. 9B) of EVs isolated by each method.
FIG. 10 shows transmission electron microscopy (TEM) images of EVs isolated by SP, the ExoQuick kit, and the exoEasy kit.
FIG. 11 depicts the results of total protein quantification and ELISA analysis for EVs isolated by two-step SP from pig plasma spiked with LNCaP-derived EVs. FIG. 11A shows the results of total protein quantification, and FIG. 11B shows the results of ELISA for EV markers.
FIG. 12 is a schematic view showing the isolation of EVs from a biological fluid by a single-step SP.
FIG. 13 shows the results of CD9/CD81 sandwich enzyme-linked immunosorbent assay (ELISA) for determining the optimal ammonium sulfate (AS) concentration for EV isolation from LNCaP culture media with various fetal bovine serum (FBS) concentrations. FIG. 13A shows the results obtained using serum-free advanced RPMI medium; FIG. 13B shows the results obtained using an RPMI medium with 5% FBS; FIG. 13C shows the results obtained using an RPMI medium with 5% exosome-free (EF)-FBS; and FIG. 13D shows the results obtained using an advanced RPMI medium with 0.1% exosome-free-FBS.
FIG. 14 depicts graphs showing the results of the analysis of EVs isolated by salt precipitation (SP) from LNCaP culture media with various FBS concentrations. FIG. 14A shows the results of total protein quantification, and FIG. 14B shows the results for EV recovery rate.
FIG. 15 depicts graphs showing the results of NTA analysis of EVs isolated from LNCaP culture media by salt precipitation (SP). Specifically, FIG. 15 shows the concentration (FIG. 15A) and size distribution (FIGS. 15B and 15C) of EVs obtained from a serum-free culture medium (FIG. 15B) and a culture medium containing 5% exosome-free (EF)-FBS (FIG. 15C).
FIG. 16 shows TEM images of EVs isolated from LNCaP culture media by SP. FIG. 16A shows the results obtained using a serum-free culture medium, and FIG. 16B shows the results obtained using a culture medium containing 5% EF-FBS.
FIG. 17 shows graphs comparing EVs isolated by precipitation methods using ammonium sulfate and sodium acetate, respectively. FIG. 17A shows the analysis of EVs by total protein quantification; FIG. 17B shows EV recovery rate; and FIG. 17C shows the results of NTA analysis.
FIG. 18 shows the characterization of EVs isolated from urine by SP. FIG. 18A is a graph showing the results of total protein quantification, and FIG. 18B is a graph showing the results for EV recovery rate.
FIG. 19 shows the characterization of EVs isolated by SP from plasma and serum from the same donor. FIG. 19A is a graph showing the results of total protein quantification, and FIG. 18B is a graph showing the results for EV recovery rate.
FIG. 20 shows the results of the scratch-wound healing assay using EVs isolated from MDA-MB231 culture media by SP and UC. FIG. 20A shows representative images of wound closure taken at 0 and 20 hours of treatment with EVs, and FIG. 20B depicts graphs showing percent wound closure versus EV dose at 5 ug/mL EV (i), 10 ug/mL EV (ii), and (iii) 20 ug/mL EV.
FIG. 21 depicts the results of PCR analysis of standard EV markers (FIG. 21A) and cancer EV markers (FIG. 21B), and shows the results of comparison between SP and other EV isolation methods (ultracentrifugation (UC), Qiagen, and Norgen) upon non-treatment (i) and treatment (ii) with proteinase-K.
FIG. 22 shows the results of comparing the recovery rate and purity of EVs isolated from plasma using SP methods with different steps.
FIG. 23 shows the Hofmeister series. The ions on the left side of the series stabilize the protein, and salts consisting of combinations of cations and anions (NH₄⁺, K⁺, Na⁺, Mg²⁺, SO₄²⁻, HPO₄²⁻, CH₃COO⁻, citrate-, Cl-) shown in red on the left side of the series were used for EV precipitation.
FIG. 24 depicts graphs showing the recovery rates (%) of EVs isolated from conditioned media of prostate cancer cells (LNCaP, FIG. 24A) and breast cancer cells (MDA-MB231, FIG. 24B) using various salts.

### Detailed Description and Preferred Embodiments of the Invention

Unless otherwise defined, all technical and scientific terms used in the present specification have the same meanings as commonly understood by those skilled in the art to which the present invention pertains. In general, the nomenclature used in the present specification is well-known and commonly used in the art.

In the present specification, salt precipitation, fractional salt precipitation, and salt fractional precipitation all have the same meaning and are abbreviated as "SP". Salt precipitation refers to a method of separating an aggregated or precipitated fraction and a supernatant by adding a salt to a biological sample.

In the present specification, "extracellular vesicles (EVs)" refers to small secretory vesicles (generally about 30 to 800 nm) that may contain nucleic acids, proteins, or other biomolecules, and include exosomes and microvesicles. They can act as a cell messenger by transporting biomolecules to various locations in living organisms or biological systems.

As used herein, the term "biological sample" includes biological fluid samples and biological tissue samples.

In one example of the present invention, it was found that an extracellular vesicle isolation method comprising salt addition and desalting steps was simpler than conventional technology, had very high isolation efficiency and purity, and could isolate extracellular vesicles very efficiently within a short time, even without comprising an ultracentrifugation step.

In particular, it was found that, while a conventional precipitation-based extracellular vesicle isolation method had a disadvantage in that an additive added for extracellular vesicle isolation is present mixed with extracellular vesicles, the salt according to the present invention had the advantage of being easily removed in a washing process for desalting, making it possible to isolate extracellular vesicles with higher purity, and could be applied to various bio-samples such as plasma, serum, urine, saliva, tissue, and cell culture media, and would also be suitable for both the preparation of large-volume extracellular vesicle samples for industrial applications and the preparation of small-volume extracellular vesicle samples required for diagnostic and therapeutic applications.

Therefore, in one aspect, the present invention is directed to a method of isolating extracellular vesicles (EVs) from a biological fluid sample, the method comprising: (a) adding a salt to the biological fluid sample to a concentration ranging from 0.1 M to a saturation concentration; (b) separating an aggregated or precipitated extracellular vesicle fraction and a supernatant from the sample to which the salt has been added; and (c) isolating extracellular vesicles by desalting the extracellular vesicle fraction separated in step (b).

In the present invention, the term "saturation concentration" refers to the concentration at which a salt is added to a sample to reach the equilibrium state of dissolution. It will be obvious to those skilled in the art that the saturation concentration may vary depending on the type of salt, type of sample, temperature, or pressure.

In the present invention, for example, when a biological sample is in a water-like state, the saturation concentration of ammonium sulfate therein may be 4.1 M at 25°C, and the saturation concentration of ammonium phosphate therein may be 3.9 M at 25°C, the saturation concentration of potassium phosphate therein may be 0.8 M at 25°C, the saturation concentration of sodium sulfate therein may be 2.0 M at 25°C, and the saturation concentration of trisodium citrate may be 3.6 M at 25°C, without being limited thereto.

In the present invention, the concentration of the added salt in the biological fluid sample may range from 0.1M to a saturation concentration, preferably 0.1 M to 4 M, more preferably 1 M to 3 M, even more preferably 1.4 M to 2.25 M, without being limited thereto.

In the present invention, step (a) may be performed by adding the salt 1 to 15 times to a final concentration ranging from 0.1 M to a saturation concentration.

That is, the extracellular vesicle isolation method according to the present invention corresponds to a single-step salt precipitation method when the salt addition step is one time, and when the step of adding the salt is performed once, and corresponds to a multi-step salt precipitation method when the step of adding the salt is performed more than once.

In the present invention, in the case of the multi-step salt precipitation method, the step of adding the salt may be repeated two or more times, preferably 2 to 15 times, more preferably 2 to 10 times, most preferably 2 to 6 times, without being limited thereto. When the step of adding the salt is repeated, the type of additional salt may or may not be the same as the type of salt added in the previous step. In addition, the salt concentration in each salt addition step may vary depending on the number of repetitions. However, as the salt addition step is repeated, the salt concentration of the sample after salt addition may increase. Multi-step salt precipitation can produce one or more separate precipitates, each of which can be processed independently to yield the same or different extracellular vesicles.

In one example of the present invention, the optimal salt concentration for EV isolation was 1.75 to 2 M for the single-step salt precipitation method, and was 1.8 to 2 M for two-step salt precipitation among the multi-step salt precipitation methods, 1.5 to 2.25 M for three-step salt precipitation, and 1.4 to 1.9 M for six-step salt precipitation.

Therefore, in the present invention, step (c) may comprise isolating extracellular vesicles from the extracellular vesicle fraction separated from the sample to which the salt has been added to a concentration of 1.4 M to 2.25 M, without being limited thereto.

In one example of the present invention, a solution containing a precipitate and a supernatant may be obtained by adding a salt to a biological sample. This solution is separated into a first fraction, defined herein as fraction-1 (F1), and a first supernatant. In the case of multi-step salt precipitation, a salt is added to the first supernatant for second precipitation, and the second fraction, fraction-2 (F2), and the second supernatant are separated from the resulting solution. Because the salt is added to the first supernatant, the salt concentration of the second solution will be higher than that of the first solution. The process of adding the salt and separating the precipitate and the supernatant is repeated until all fractions are separated. Salt precipitation may also be performed on resuspended precipitates.

In the present invention, the salt may include a kosmotrope (kosmotropic ion) or a chaotrope (chaotropic ion). Kosmotrope refers to a salt that stabilizes the protein structure, and chaotrope refers to a salt that destabilizes the protein structure (Wiggins, P. M. (2001). Cellular and Molecular Biology, 47, 735-744).

In the present invention, the salt may contain a polyvalent anion and a monovalent cation.

As used herein, the term "polyvalent anion" refers to an anion with a valence of 1 or more, and the term "monovalent cation" refers to a cation with a valence of 1.

Specifically, the polyvalent anion may be sulfate, phosphate, or citrate, and the monovalent cation may be an ammonium ion, a potassium ion, or a sodium ion, without being limited thereto.

In one embodiment of the present invention, the salt may be ammonium sulfate, ammonium phosphate, potassium phosphate, sodium sulfate, or trisodium citrate, without being limited thereto.

In the present invention, the salt may be in a solid or liquid form. In the present invention, the salt in a liquid form may have the same meaning as the term "salt solution".

In the present invention, the salt may be a watersoluble salt or a water-insoluble salt, preferably a watersoluble salt, without being limited thereto. Water-insoluble salts (e.g., calcium citrate and/or other salts with low solubility in water) with chelating agents (e.g., EDTA) that improve the solubility of insoluble salts may also be used. High concentrations of the salt are useful when the concentration of extracellular vesicles in the sample is low.

In one embodiment of the present invention, a variety of buffers commonly used for biological samples may be used for processing of the extracellular vesicle sample to which the salt has been added, and include phosphate, acetate, citrate and TRIS buffers. The pH of the buffer may be any pH that is compatible with the sample, and is from 6 to 8, without being limited thereto.

In the present invention, step (b) may be performed by sedimentation, filtration, or centrifugation.

In the present invention, the centrifugation may be performed at 2,000×g to 15,000xg for 5 to 30 minutes, without being limited thereto.

In the present invention, biological fluid samples may be processed at a temperature of -5 to 40°C, but are not limited thereto, and any temperature conditions may be applied without limitation, as long as they do not result in the disruption of extracellular vesicles.

In one example of the present invention, plasma was maintained at room temperature (RT), and a salt solution was added thereto at room temperature to obtain a first precipitate and supernatant. The first precipitate and supernatant are separated by filtration at room temperature or centrifugation at >10,000xg at room temperature or 4°C.

In the present invention, the desalting may be performed using filtration, centrifugation, dialysis, reverse osmosis, or a desalting column, without being limited thereto.

In one embodiment of the present invention, when a salt is added to a biological sample and sedimentation, filtration, or centrifugation is performed, the sample is separated into an aggregated or precipitated fraction and a supernatant. Since the extracellular vesicles contained in the precipitated fraction are the final targeted to be isolated by the method according to the present invention, they are referred to as the aggregated or precipitated extracellular vesicle fraction.

The precipitated extracellular vesicles may be separated using centrifugation, filtration, or other methods. For small scales, centrifugation is preferable, and for large scales, filtration is preferable. When centrifugation is used, the precipitate forms a pellet from which the supernatant can be removed by pipetting. When filtration is used, the precipitate forms a cake on filter paper, and the filtrate is the supernatant.

The precipitate may be dissolved in buffer and subjected to additional processes, including desalting, further fractionation, chromatography, etc., or a combination thereof, to purify the isolated extracellular vesicles. The method may further comprise a centrifugal tangential flow filtration or ultrafiltration step of removing salt, small proteins and lipoproteins using an ultrafiltration instrument equipped with a filter with a pore diameter size of 10 to 100 nm or a polymer-based filter with an MWCO of 100 kD to 300 kD.

Centrifugal filtration and/or ultrafiltration can remove salt, low-molecular-weight species, and/or processing agents. Centrifugal filtration or ultrafiltration may be performed between salt precipitation steps and/or chromatography steps, and/or between a salt precipitation step and a chromatography step.

In the present invention, an ultrafiltration step, an affinity chromatography step, or a density gradient ultracentrifugation step may be further performed on the extracellular vesicle fraction, without being limited thereto.

In the present invention, although not necessary, high-purity extracellular vesicles may be obtained if the biological fluid sample is clarified before salt precipitation, to remove any debris from the sample. Methods of clarification include, but are not limited to, centrifugation, ultracentrifugation, filtration, or ultrafiltration.

In the present invention, in order to obtain high-purity extracellular vesicles, extracellular vesicles isolated by salt precipitation may be further purified based on their size, density, or proteins exposed on the surface of the extracellular vesicle.

Extracellular vesicles may be further fractionated using conventional methods such as chromatography or ultracentrifugation with or without the use of a density gradient. Purification of extracellular vesicles from the fraction obtained using the salt may require purification steps including chromatography, or may require purification steps other than chromatography. Sub-populations of extracellular vesicles may also be isolated by using other properties of the extracellular vesicles such as the presence of surface markers. Surface markers that may be used for fractionation of extracellular vesicles include, but are not limited to, tumor markers and MHC class II markers. Other surface markers associated with extracellular vesicles include CD9, CD81, CD63 and CD82 (Thery et al. Nat. Rev. Immunol. 2 (2002) 569-579; Valadi et al. Nat. Cell. Biol. 9 (2007) 654-659).

To obtain pure extracellular vesicles, chromatography including, but not limited to, size-exclusion, cation-exchange, anion-exchange, hydrophobic interaction, or affinity chromatography may be performed. A single chromatography process (e.g., affinity chromatography or size-exclusion chromatography) may be performed. A series of the same chromatography process or different chromatography processes may be performed in sequence (e.g., two ion-exchange chromatography runs in sequence, or an affinity chromatography run followed by a size-exclusion chromatography run). A series of processes may be performed (e.g., salt precipitation followed by an affinity chromatography run followed by a hydrophobic interaction chromatography run). A series of the different chromatography processes with intermediate processes may be performed in sequence (e.g., an affinity chromatography run followed by an ion exchange chromatography run), and a series of different chromatography processes with intermediate processes may be performed (e.g., an affinity chromatography run followed by salt precipitation followed by hydrophobic interaction chromatography run). Alternatively, a combination of any of these schemes may be performed.

Examples of a method to obtain pure extracellular vesicles using surface molecules include a method in which extracellular vesicles having tetraspanin markers (CD9, CD63, and CD81) on their surface are isolated using antibody-coated magnetic particles. For example, Dynabeads (superparamagnetic beads with a diameter of 1 to 4.5 µm) may be conjugated with a cocktail of anti-human CD9 antibody, anti-human CD63 antibody, and anti-human CD81 antibody, either directly to the bead surface or via a secondary linker (e.g., anti-mouse IgG). Antibody-coated Dynabeads may be added to an extracellular vesicle sample prepared using a salt and incubated at 2 to 8°C or at room temperature from 0 minutes to overnight. Dynabeads with bound extracellular vesicles may then be collected using a magnet. The isolated bead-bound extracellular vesicles may then be resuspended in an appropriate buffer, such as phosphate buffered saline (PBS), and used for downstream analysis (reverse transcription real-time polymerase chain reaction (RT-qPCR), sequencing, Western blotting, flow cytometry, etc.). Similar protocols may be used for any other surface marker for which an antibody or other specific ligand is available. Indirect binding methods such as those using biotin-avidin may also be used.

Once extracellular vesicles have been isolated from the sample, the contents of the extracellular vesicles may be extracted for study and characterization. Biological materials which may be extracted from extracellular vesicles include proteins, peptides, RNA, DNA, lipids, and the like. For example, the miRNeasy kit (217004, Qiagen) may be used to recover DNA and RNA from extracellular vesicles. Additionally, the miRNeasy kit (217004, Qiagen) may be used to recover total RNA from extracellular vesicles. Likewise, the mirVana^{™} PARIS Kit (AM1556, Life Technologies) may be used to recover native protein and RNA species, including small RNAs such as miRNA, snRNA, and snoRNA, from extracellular vesicles.

As used herein, the term "biological fluid" refers to any fluid isolated or derived from an organism, including prokaryotes, eukaryotes, bacteria, fungi, yeast, invertebrates, vertebrates, reptiles, fish, insects, plants, and animals, and includes, but is not limited to, serum, plasma, whole blood, urine, saliva, breast milk, tears, sweat, joint fluid, cerebrospinal fluid, semen, vaginal fluid, sputum, pleural fluid, lymph fluid, ascitic fluid, and amniotic fluid. Bronchial lavage fluid and media taken from cultured cells (e.g., cell culture supernatant, conditioned media, cell media, or cell culture media) may also be a biological fluid.

In the present invention, extracellular vesicles may be isolated directly from biological samples by the salt precipitation method. Alternatively, extracellular vesicles may be isolated or enriched by the salt precipitation method from extracellular vesicle-containing solutions prepared by other methods such as ultracentrifugation, density gradient ultracentrifugation, size-exclusion chromatography (SEC), tangential flow filtration (TFF) or precipitation.

In another aspect, the present invention is directed to a method of isolating extracellular vesicles (EVs) from a biological tissue sample, the method comprising: (a) lysing or grinding and clarifying the biological tissue sample; (b) adding a salt to the clarified sample to a concentration ranging from 0.1 M to a saturation concentration; (c) separating an aggregated or precipitated extracellular vesicle fraction and a supernatant from the sample to which the salt has been added; and (d) isolating extracellular vesicles by desalting the extracellular vesicle fraction separated in step (c).

In the present invention, the concentration of the added salt in the sample may range from 0.1 M to a saturation concentration, preferably 0.1 M to 4 M, more preferably 1 M to 3 M, even more preferably 1.4 M to 2.25 M, without being limited thereto.

In the present invention, step (b) may be performed by adding the salt 1 to 15 times to a final concentration ranging from 0.1 M to a saturation concentration.

In the present invention, step (d) may comprise isolating extracellular vesicles from the extracellular vesicle fraction separated from the sample to which the salt has been added to a concentration of 1.4 M to 2.25 M, without being limited thereto.

In the present invention, the salt may contain a polyvalent anion and a monovalent cation.

Specifically, the polyvalent anion may be sulfate, phosphate, or citrate, and the monovalent cation may be an ammonium ion, a potassium ion, or a sodium ion, without being limited thereto. In one embodiment of the present invention, the salt may be ammonium sulfate, ammonium phosphate, potassium phosphate, sodium sulfate, or trisodium citrate, without being limited thereto.

The salt may be in a solid or liquid form.

In the present invention, step (c) may be performed by sedimentation, filtration, or centrifugation.

In the present invention, the desalting may be performed using filtration, centrifugation, dialysis, reverse osmosis, or a desalting column.

In the present invention, an ultrafiltration step, an affinity chromatography step, or a density gradient ultracentrifugation step may be further performed on the extracellular vesicle fraction.

In the present invention, the method of isolating extracellular vesicles from the biological tissue sample comprises the same steps as those of the method of isolating extracellular vesicles from the biological fluid sample, except that the sample is biological tissue rather than a biological fluid, and thus the method of isolating extracellular vesicles from the biological tissue sample further comprises the step of lysing or grinding and clarifying the biological tissue sample and incubating the sample. In the method of isolating extracellular vesicles from the biological tissue sample, the description of contents that overlap with the method of isolating extracellular vesicles from the biological fluid sample, such as the type of salt, salt concentration, and repetitions of the salt addition step, will be omitted.

As used herein, the term "biological tissue" means a collection of cells from prokaryotes, eukaryotes, bacteria, fungi, yeast, invertebrates, vertebrates, reptiles, fish, insects, plants or animals. In addition, cultured cells may be a biological tissue. Non-limiting examples of the biological tissue samples include surgical samples, biopsy samples, tissues, feces, plant tissue, insect tissue, and cultured cells.

When isolating extracellular vesicles from tissue sources, the method may further comprise a step of homogenizing the tissue in order to obtain a single cell suspension, followed by lysis or grinding of the cells to release the extracellular vesicles. In this case, it is important to select homogenization and lysis/grinding procedures that do not result in disruption of the extracellular vesicles.

In the present invention, incubation of the sample to which the salt has been added may be performed in any time range, generally 1 second to 24 hours, more generally 5 minutes to 12 hours. Incubation time is affected by, *inter alia,* salt concentration, incubation temperature, extracellular vesicles, and other components of the sample.

In still another aspect, the present invention is directed to a kit for isolating extracellular vesicles (EVs) comprising a salt and a buffer.

In the present invention, the salt may contain a polyvalent anion and a monovalent cation. Specifically, the polyvalent anion may be sulfate, phosphate, or citrate, and the monovalent cation may be an ammonium ion, a potassium ion, or a sodium ion, without being limited thereto. In one embodiment of the present invention, the salt may be ammonium sulfate, ammonium phosphate, potassium phosphate, sodium sulfate, or trisodium citrate, without being limited thereto.

The salt may be in a solid or liquid form, without being limited thereto.

In the present invention, the kit may be used to isolate extracellular vesicles from a biological fluid or biological tissue. Therefore, the description of contents that overlaps with the contents described above with respect to the method of isolating extracellular vesicles will be omitted.

In the present invention, the kit may further comprise, but is not limited to: (i) a vessel containing an antibody or ligand that binds to a surface marker exposed on the surface of the extracellular vesicle or to a protein present inside the extracellular vesicle; (ii) at least one solid support that binds directly or indirectly to a surface marker exposed on the surface of the extracellular vesicle or to a protein present inside the extracellular vesicle; and/or (iii) a vessel containing at least one salt and at least one buffer for performing density gradient centrifugation of the extracellular vesicles.

In the present invention, the surface marker may be selected from the group consisting of HLA DP haplotypes, HLA DQ haplotypes, HLA DR haplotypes, CD9, CD81, CD63, and CD82, without being limited thereto.

In the present invention, the solid support may be resin or beads, without being limited thereto.

In the present invention, the beads may be silica, magnetic particles, polystyrene, or agarose.

Hereinafter, the present invention will be described in more detail with reference to examples. These examples are only for illustrating the present invention, and it will be obvious to those skilled in the art that the scope of the present invention should not be construed as being limited by these examples.

### Example 1: Materials and Methods

### Example 1-1: Preparation of Salt Stock Solutions

For example, 100 mL of a stock solution containing 2 M ammonium sulfate (AS) in DI water may be prepared by dissolving 30.9 g of AS in 50 mL of DI water. After adjusting the pH to a desired value, additional DI water may be used to bring the solution volume to 100 mL. To prepare 100 mL of a stock solution containing 4 M AS in DI water, 75 g of AS is added to 40 mL of DI water, the pH of the solution is adjusted, and then water is added to make the final volume 100 mL. Solutions containing 1 to 4 M AS with or without PBS or other buffers or NaCl may be prepared in a similar manner. AS stock solutions may be stored at room temperature for a long period of time.

### Example 1-2: Preparation of Biological Fluid Samples

Samples are removed from storage and placed on ice. If the biological fluid is in a frozen state, it may be thawed slowly at room temperature or in lukewarm water until the sample is completely liquid. Samples may be stored on ice until needed. The samples may be centrifuged at 2,000×g for 30 minutes to remove cell debris. Next, the supernatant containing the cell-free/debris-free sample may be transferred to a fresh container and held on ice until precipitation. For extracellular vesicle precipitation, 100 µL to 1 mL (or other preferred volume) of a cell-free sample may be transferred to a new tube and combined with the desired volume of salt precipitation reagent.

For example, to achieve a 1 M final concentration of a salt, 33 µL of 4 M stock may be added to 100 µL of serum. The serum/reagent mixture may then be mixed well either by vortexing or pipetting up and down until there is a homogenous solution. (Solution may have a cloudy appearance). The samples may then be incubated at room temperature for 5 minutes to 2 hours. After incubation, the samples may be centrifuged at room temperature or 4°C at 2,000×g to 10,000xg for 5 to 30 minutes. The supernatant is aspirated and discarded. Extracellular vesicles will be contained in the pellet at the bottom of the tube. The pellet may be resuspended in a convenient volume of phosphate buffered saline (PBS), e.g., 50 µL for 100 µL serum input. The pellet may be difficult to resuspend, and in this case, a pipet tip may be used to completely resuspend the pellet in the solution. Alternatively, the pellets may be incubated for 30 minutes at 37°C, and then vortexed. Once the pellet has been resuspended, it may be stored at 4°C (short term) or at - 20°C (long term). Solutions containing AS at 1 to 4M final concentration (when mixed with serum sample), with or without PBS buffer or NaCl, may be used in a similar fashion, with serum input typically ranging from several microliters to several milliliters.

### Example 1-3: Cell Culture and Preparation of Culture Supernatants

LNCaP cells purchased from ATCC were cultured in (1) Roswell Park Memorial Institute medium (RPMI, Gibco, Thermo Fisher Scientific) supplemented with 1% antibiotics/antimycotics, (2) RPMI supplemented with 5% fetal bovine serum (FBS) and 1% antibiotics/antimycotics, (3) RPMI supplemented with 5% Exo-Free FBS (ef-FBS, Systems Biosciences Inc., CA, USA) and 1% antibiotics/antimycotics, or (4) Advanced RPMI (Gibco, Thermo Fisher Scientific) supplemented with 0.1% ef-FBS and 1% antibiotics/antimycotics. The cells were cultured at 37°C with 5% CO₂. Cell culture media were collected after 48 hours of culture, centrifuged at 300g for 10 minutes at 4°C to remove dead cells, and then spun at 2,000g for 15 minutes to completely remove dead cells and cell debris. The supernatants were stored at -80°C until use.

### Example 1-4: CD9-CD81 Sandwich ELISA

All samples were prepared to keep the same input volume in all comparative analyses. A 96-well plate (Corning Inc., NY, USA, cat#3590) was coated with 50 µL of coating antibody (10 ug/mL anti-CD9 in PBS buffer; MEM 61; Abcam, Cambridge, UK) and incubated overnight at 4°C. The next morning, the plate was blocked with 1% bovine serum albumin (BSA)-PBS buffer for 1 hour at 37°C. After washing with 0.1% BSA-PBS buffer (washing buffer), the plate was further incubated with the extracellular vesicle solution in PBS buffer (50 µL) for 2 hours at room temperature. After removing the solution, the plate was washed twice with washing buffer and then incubated with a biotin-conjugated secondary antibody (anti-CD81; LifeSpan Biosciences, Inc., Seattle, WA; USA) in PBS buffer (50µL; 500 ng/mL) for 1 hour at room temperature. After washing three times with washing buffer, the plate was incubated with an HRP-conjugated streptavidin solution in PBS buffer (50 µL; 1:500) at room temperature for 30 minutes and then washed three times with washing buffer. Then, TMB solution (50 µL) was added, the plate was incubated at room temperature for 15 minutes, and then 50 µL of stop solution was added to each well. Solution absorbance was measured using a plate reader spectrophotometer (TECAN, Morrisville, NC, USA) at 450 nm. A standard curve for CD9-CD81 ELISA was prepared using the initial sample, and EV recovery (%) was calculated relative to the initial sample.

### Example 1-5: Sandwich ELISA for Albumin and Lipoprotein Markers

Commercial duo sets, Human Serum Albumin DuoSet ELISA (DY1455) and Human Apolipoprotein A-I/ApoA1 DuoSet (DY3664-05), obtained from R&D Systems, were used for albumin and apo AI analyses, respectively, according to the manufacturer's instructions.

### Example 1-6: Direct ELISA for ALIX Detection

Extracellular vesicles (EVs) were lysed using RIPA buffer containing 1% proteinase inhibitor for 30 minutes on ice with gentle vortexing at 10-minute intervals. Each well of a 96-well plate (Corning Inc., NY, USA, cat#3590) was coated with a 50 µL volume of EV lysate (diluted 1:50 in PBS) and incubated overnight at 4°C. The next morning, the plate was blocked with 1% bovine serum albumin (BSA)-PBS buffer for 1 hour at room temperature. After washing with 0.1% BSA-PBS buffer (washing buffer), the plate was loaded with anti-ALIX antibody (Abcam, Cambridge, UK) in PBS buffer (50 µL; 500 ng/mL) and incubated for 1 hour at room temperature. After washing three times with washing buffer, the plate was incubated with a solution of HRP-conjugated detection antibody in PBS buffer (50 µL) at room temperature for 20 minutes and then washed three times with washing buffer. TMB solution (50 µL) was added, and the plate was again incubated at room temperature for 15 minutes. Then, 50 µL of stop solution was added to each well. Solution absorbance was measured using a plate reader spectrophotometer at 450 nm.

### Example 1-7: Quantification and Sizing of Extracellular Vesicles Using NanoSight NS500 Instrument

Extracellular vesicles purified from a fluid sample by salt precipitation were quantified and sized using an NS500 instrument (NanoSight, UK) according to the manufacturer's protocol. The image analysis NTA software allows the user to automatically track and size nanoparticles on an individual basis. The isolated extracellular vesicle sample was vortexed and diluted with PBS filtered through a 200-nm filter to obtain the recommended 25 to 100 particles/frame of the NTA system. All measurements were performed under identical settings to ensure consistent results. Each sample was analyzed three times, and mean values were plotted.

### Example 1-8: Transmission Electron Microscopy (TEM)

300-mesh formvar and carbon-coated copper grids (Electron Microscopy Science, PA) were coated with 0.1% poly-L-lysine (Sigma-Aldrich) for 30 minutes at room temperature, and then incubated with the extracellular vesicle sample, appropriately diluted cells in PBS buffer, at room temperature for 30 minutes. Next, the extracellular vesicles on the grids were fixed with a 4% paraformaldehyde solution at room temperature for 10 minutes, and then washed sequentially with PBS and DI water. The grids were stained with UranyLess and imaged with a JEM-2100 transmission electron microscope (JEOL, Japan).

### Example 1-9: RNA Extraction and Reverse Transcription Polymerase Chain Reaction (RT-qPCR)

To analyze gene expression, total RNA was extracted from EVs isolated by various methods from plasma spiked with LNEVs (EVs obtained from LNCaP culture media) using the miRNeasy kit (Qiagen). cDNA was prepared using a SuperScript VILO cDNA synthesis kit (Thermo Fisher Scientific). Real-time PCR was performed using the gene-expression master mix kit (Thermo Fisher Scientific) and Taqman probe with a QuantStudio 6 real-time PCR instrument (Thermo Fisher Scientific) using the following conditions: 50°C for 2 min, 95°C for 10 min, followed by 40 cycles, each consisting of 95°C for 15 sec and 60°C for 30 sec. All samples were analyzed in triplicate, and data are presented as mean ± standard deviation (SD).

### Example 2: Isolation of Extracellular Vesicles (EVs) by Multi-Step Salt Fractional Precipitation Method

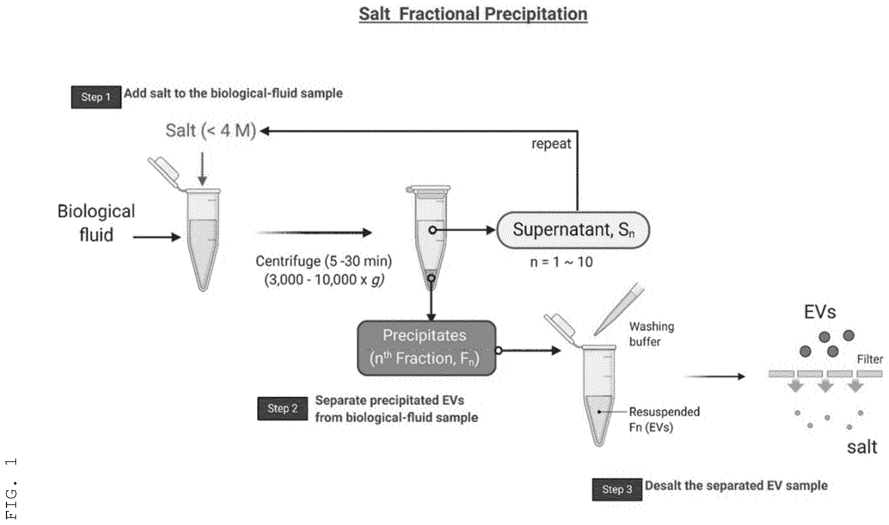

FIG. 1 shows a schematic view of a multi-step salt separation and precipitation method of isolating extracellular vesicles from a biological fluid sample.

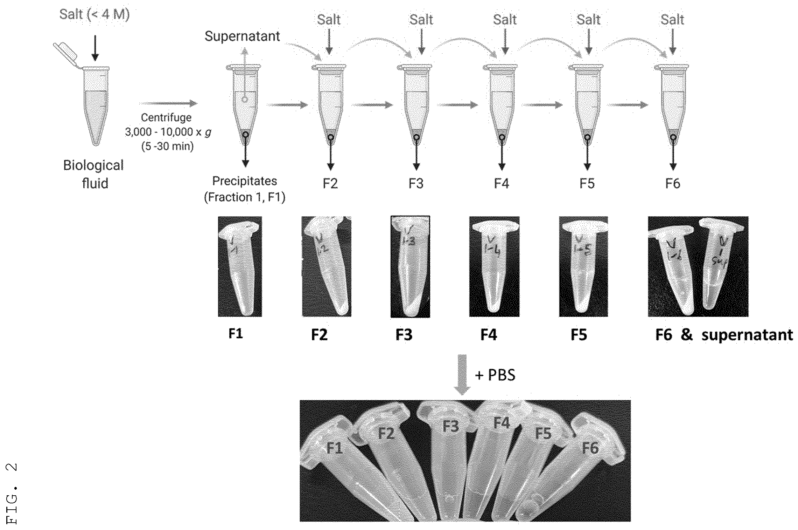

In this Example, 1 mL of fresh frozen plasma was used as a biological fluid sample. A plasma sample was incubated with increasing amounts of ammonium sulfate (AS), and F1 to F6 fractions were collected, as shown in FIG. 2. Each fraction was analyzed to determine EV and protein contents. The multi-step salt fractional precipitation (SP) method includes the following steps:

Step (1) of adding solid AS to the biological fluid to produce the first precipitate (Fraction-1, F1), wherein the AS concentration of the solution is 0.45 M; step (2) of separating the first precipitate by centrifugation at >10000xg at room temperature to produce a first supernatant; step (3) of adding AS to the first supernatant to produce a second precipitate (Fraction-2, F2), wherein the concentration of AS in the solution is 0.95 M; and step (4) of separating the second precipitate to produce a second supernatant, and repeating the steps of adding AS so that the concentrations of AS are 1.4, 1.9, 2.35, and 2.8 M in F3, F4, F5, and F6, respectively, and centrifuging the precipitates until all desired fractions are collected. The combination of these steps is referred to as "SP". Precipitates F1 to F6 are resuspended in buffer and desalted using a centrifugal filtration device (FIG. 2).

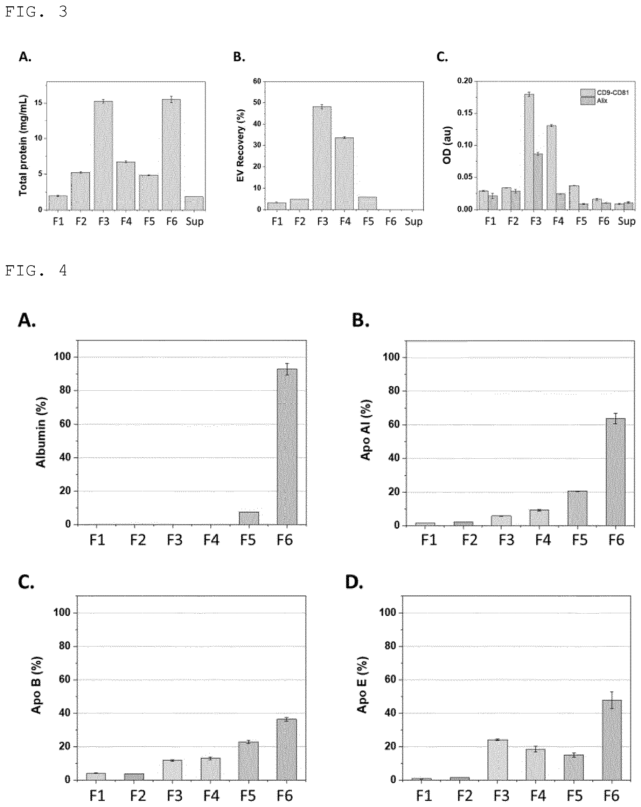

These fractions, along with the final supernatant, were analyzed by bicinchoninic acid assay (BCA) for total protein quantification. Enzyme-linked immunosorbent assay (ELISA) for qualitative analysis of EVs was performed using both EV standard markers and non-EV markers (FIG. 3). Markers for lipoproteins, Apo B, Apo E, and Apo AI were expressed at different levels in all the fractions (FIG. 4).

As a result of assessing the contents of the fractions by ELISA for EV and non-EV markers, most CD9-CD81 positive EVs were found in F3 and F4, and most ALIX-containing EVs were found in F3 (FIG. 3). ELISA for albumin shows that most albumin precipitates in later fractions F5 and F6. Markers for lipoproteins, Apo B, Apo E, and Apo AI, appeared at different levels in all the fractions, but were the highest in F5 and F6 (FIG. 4).

### Example 3: Isolation of Extracellular Vesicles (EVs) from Plasma by Three-Step Salt Fractional Precipitation Method

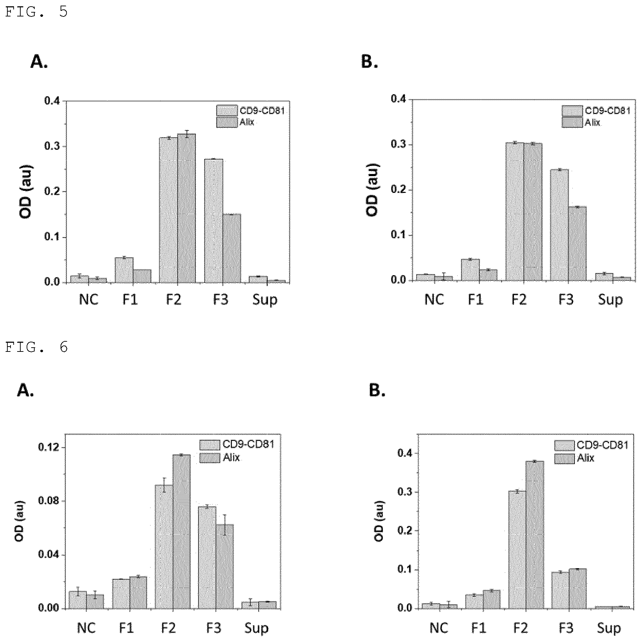

An experiment was conducted by collecting EVs, recovered in F3 and F4 in the six-step salt fractional method described in Example 2, as one fraction, and reducing the number of fractions to three to remove protein impurities as much as possible. SP was performed with solid or liquid AS using 1 mL of a biological fluid sample (fresh frozen plasma). The plasma was mixed with a saturated solution of liquid AS or solid AS, making sure the resulting solutions contained 0.75, 1.5, and 2.25 M of AS in F1, F2, and F3, respectively. The precipitate and supernatant were separated by centrifugation. The step of adding AS and the step of separating the precipitate from the supernatant by centrifugation were continued until three precipitate fractions (F1 to F3) and the supernatant were collected. EV contents were analyzed by ELISA using EV markers. Most of the CD9-CD81 and ALIX positive EVs were found in F2 and F3 of the solid and liquid SP methods (FIG. 5). Therefore, it can be seen that AS in solid or liquid form can be used in SP without any difference in performance.

### Example 4: Scalability Evaluation

Three-step salt fractional precipitation was performed to isolate EVs from different volumes of plasma, specifically 0.4 mL and 4 mL of plasma. Liquid salt was used, and the three precipitate fractions and the supernatant were collected using the method described in Example 3. The results of EV marker ELISA of EVs isolated from the two samples were similar, and most EVs were found in F2 and F3 in both cases (FIG. 6). This means that, unlike the filtration or size-exclusion chromatography method that can only be applied to small-volume samples, the multi-step salt precipitation method can be applied both on a small scale for diagnostic purposes and on a large scale for industrial applications.

### Example 5: Isolation of EVs from Plasma Samples by Two-Step Salt Fractional Precipitation Method

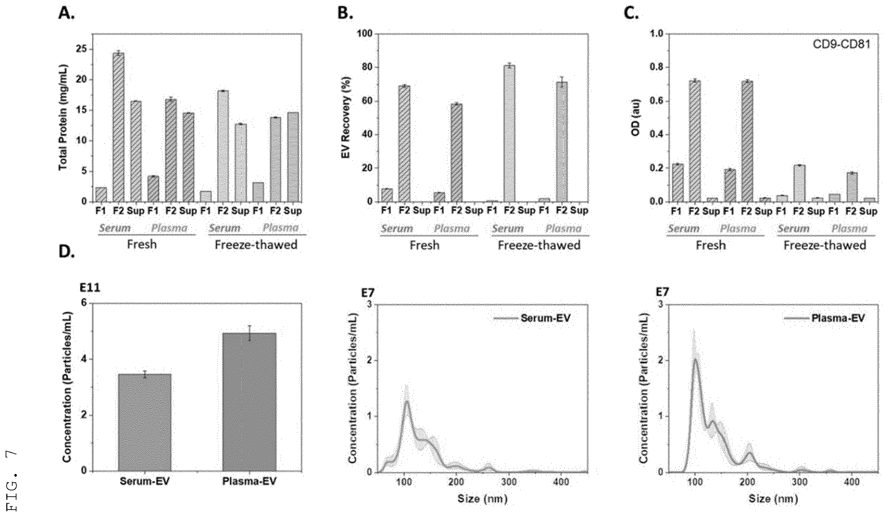

In order to enable EVs collected in F2 and F3 in the three-step salt fractional precipitation method shown in Example 3 to be collected in one fraction, EVs from 1 mL of each of (1) fresh serum, (2) freeze-thawed serum, (3) fresh plasma, and (4) freeze-thawed plasma were enriched in one fraction (F2) by two-step SP. The major components of plasma include fibrinogen, globulin, and albumin. Fibrinogen is known to precipitate at 0.85 M AS, globulins are known to precipitate at about 1.5 M AS, and albumin is known to precipitate at ≥2.5 M AS. Therefore, in this two-step salt fractional precipitation method, fibrinogen and albumin were selectively removed, and most EVs were collected in F2. In the first step, fibrinogen was removed by adding 0.85 to 1.0 M AS, and EVs were collected at 1.8 to 2 M concentrations of AS, whereas albumin remained in the supernatant. There was no significant difference between the serum and plasma samples. Similarly, no differences were observed in EVs isolated from the fresh and freeze-thawed samples (FIG. 7). The precipitate and supernatant were analyzed for their EV content by BCA, ELISA, and NTA. EV markers were shown to be high in F2 of all the samples.

### Example 6: Comparison of Extracellular Vesicle (EV) Recovery by Two-Step Salt Precipitation (SP) and Other Commercially Available Methods

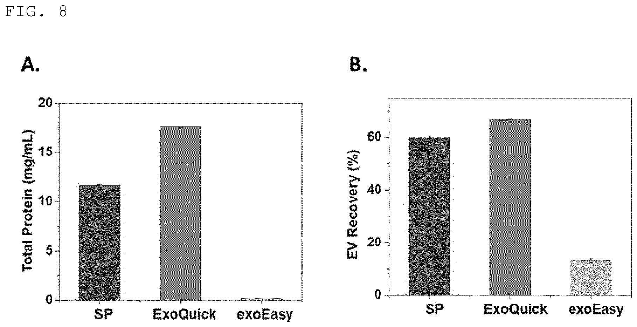

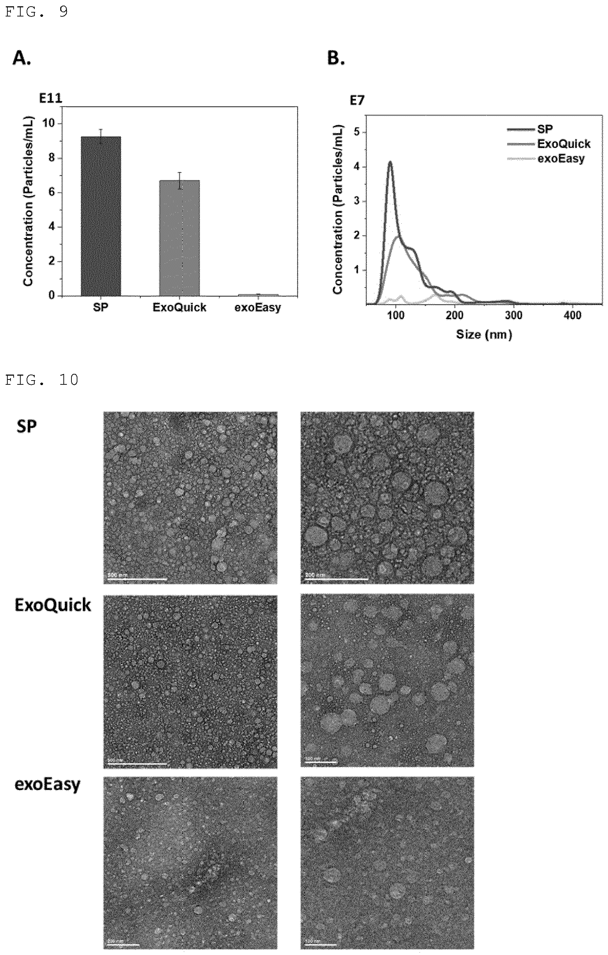

EVs were isolated from 1 mL of plasma by two-step SP. In addition, EVs were isolated from 1 mL of plasma using the ExoQuick plasma kit (EQ; Systems Biosciences), and EVs were isolated from 1 mL of plasma using the exoEasy kit (Qiagen). EVs in Fraction-2 were characterized by BCA, ELISA for EV markers, and the number of particles determined by NTA. As a result, it was shown that the SP method isolated the largest amount of EVs with a particle number concentration of 9.25 × 10¹¹ ± 8.46 × 10¹⁰ particles/mL, and exhibited a relatively small total protein amount and a similar EV recovery (%) when compared with EQ that exhibited a particle number concentration of 6.7 × 10¹¹ ± 1 × 10¹⁰ particles/mL. ExoEasy exhibited a relatively small particle number (1.05 × 10¹⁰ ± 1.44 × 10⁹ particles), the smallest total protein amount, and a low EV recovery (%) (FIGS. 8 and 9) .

Referring to transmission electron microscopy images, it appears that the three methods all showed morphologically similar vesicles, but the exoEasy kit showed a smaller particle size than SP and EQ (FIG. 10).

### Example 7: Isolation of Extracellular Vesicles (EVs) from Pig Plasma

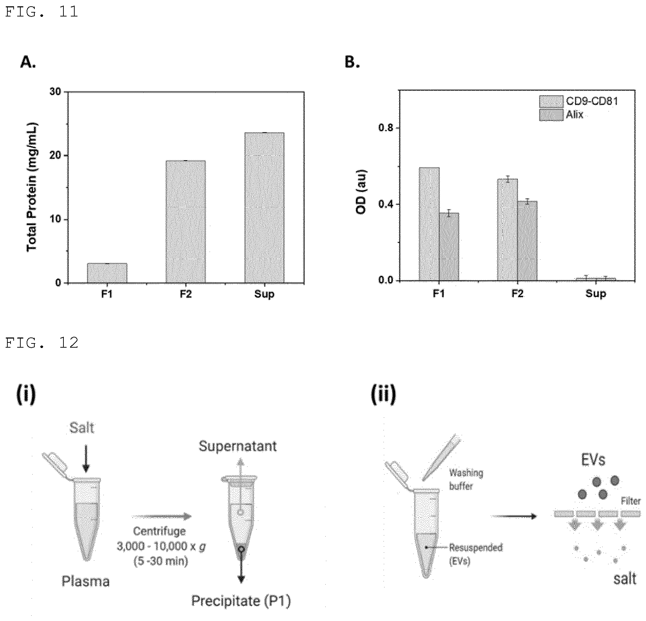

EVs were isolated from 1 mL of pig plasma containing LNCaP cell-derived EVs. Two-step salt fractional precipitation was used, and fractions were analyzed for their EV content by BCA and ELISA assay for LNCaP EV markers (FIG. 11). EVs were isolated from the first and second fractions of salt precipitation. This means that the salt precipitation method can be applied to isolate EVs from various biological fluid samples, such as human blood or pig blood.

### Example 8: Isolation of EVs by One-Step Salt Precipitation with Ammonium Sulfate

FIG. 12 is a schematic diagram illustrating isolation by one-step salt precipitation, which is the simplest form of multi-step salt precipitation.

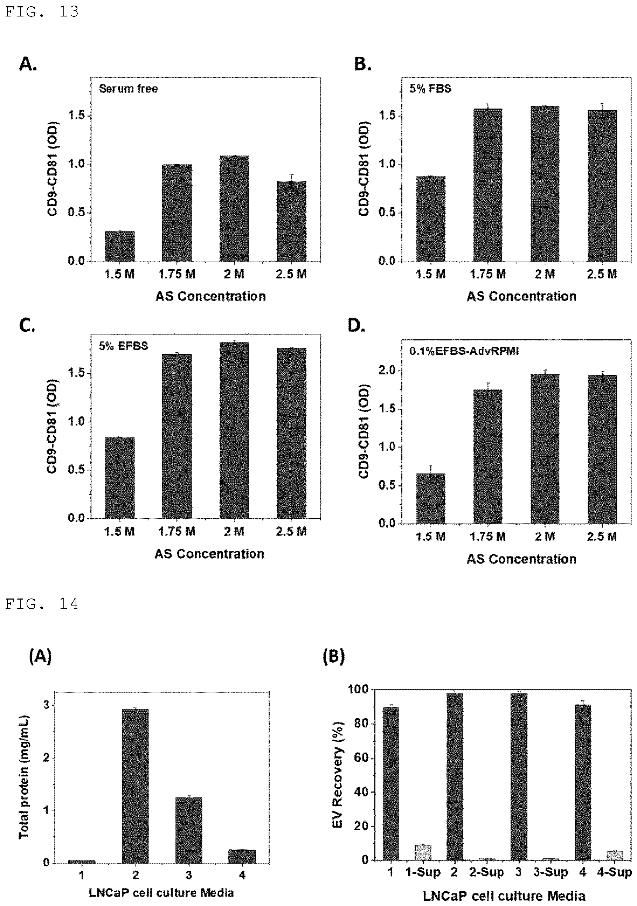

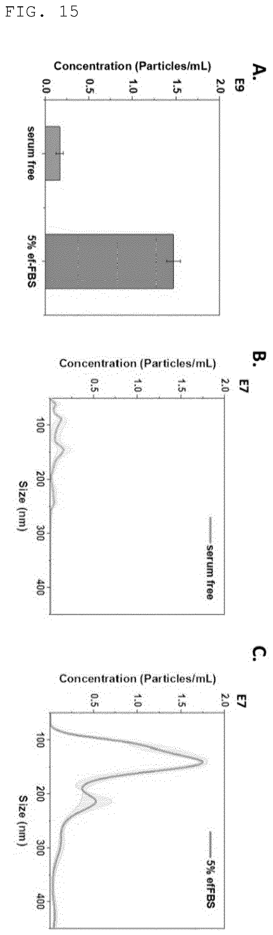

An experiment was first conducted to find the optimal AS concentration for EV isolation by the single-step precipitation method from each of (1) serum-free LNCaP culture media (LN-CM), (2) LN-CM containing 5% FBS, (3) LN-CM containing 5% ef-FBS, and (4) LN-CM containing 0.1% ef-FBS in advanced RPMI. Saturated solutions of AS were added to 1 mL of LN-CM to final concentrations of 1.5, 1.75, 2, and 2.5 M, respectively, after mixing. The contents of the tube were mixed by inverting the tube 5 or 6 times, and the separated precipitates were collected by centrifugation at 10,000xg for 10 minutes. Collected EVs were assessed by CD9-CD81 sandwich ELISA (FIG. 13). Most EVs from all of the samples were isolated at a final concentration of 1.75 to 2 M, regardless of the amount of FBS present in the media. Additionally, EVs isolated at an AS concentration of 2 M were analyzed by BCA, ELISA, NTA, and TEM. Due to the lack of contaminating proteins or other particles in FBS, EVs obtained from serum-free media had the lowest protein amount and total particle number. The recovery rate of EVs was 90% for all of the four samples tested (FIGS. 14 to 16). As a result, it could be seen that, in the case of the one-step salt fractional precipitation method using an AS concentration of 1.75 to 2M, it could be seen that most (90% or more) of EVs were present in the precipitate regardless of the type of media used.

### Example 9: Comparison of EV Recovery Rates by Ammonium Sulfate and Sodium Acetate

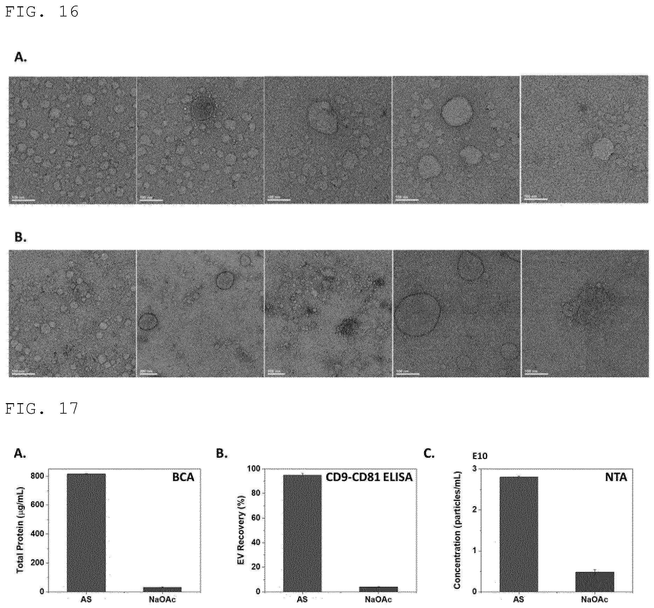

EVs were isolated from 1 mL of LN-CM containing 5% ef-FBS using each of ammonium sulfate and sodium acetate according to a previously reported protocol (J. Immunol. Met., 2014, 407, 120). For EV isolation using ammonium sulfate, LN-CM was mixed with a saturated AS solution with a final AS concentration of 1.8 M. EVs obtained in both methods were characterized by BCA, ELISA for EV markers, and the number of particles determined by NTA. As a result, compared to the sodium acetate method, which yielded 4.85 × 10⁹ ± 5.7 × 10⁸ particles/mL and an EV recovery rate of 4%, the ammonium sulfate method isolated a much larger amount of EVs with a particle number concentration of 2.8 × 10¹⁰ ± 3.06 × 10⁸ particles/mL with an EV recovery rate of >95% (FIG. 17).

### Example 10: EV Isolation from Urine by Ammonium Sulfate

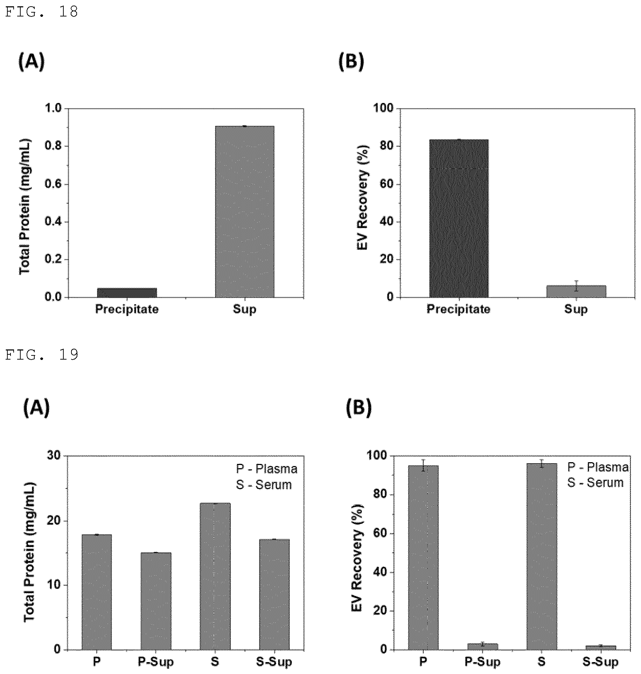

A saturated solution of AS was added to 1 mL of urine to a final AS concentration of 2 M, and EVs were isolated by the single-step salt fractional precipitation method. The contents of the tube were mixed by inversion and then immediately the precipitate was separated by centrifugation at 10,000xg for 10 min. The precipitate and supernatant were characterized by BCA assay for total protein quantification and ELISA for EV markers. As shown in FIG. 18, most (80% or more) of EVs were recovered at an AS concentration of 2 M.

### Example 11: EV Isolation from Plasma and Serum by Ammonium Sulfate

A saturated solution of AS was added to 1 mL each of plasma and serum to a final AS concentration of 2 M. The contents of the tube were mixed by inverting the tube several times, and the precipitate was collected by centrifugation at 10,000xg for 10 minutes. The precipitate and supernatant were characterized by total protein quantification and ELISA for EV markers. As a result, it was found that most of EVs were recovered along with a large amount of total protein at an AS concentration of 2 M (FIG. 19). Proteins in plasma and serum can be selectively classified according to their charge, size, and shape.

The one-step salt precipitation method exhibited an EV recovery rate of 90% or more regardless of the type of sample, such as plasma or serum. Although a large amount of impurity proteins is also isolated, if the substance to be finally detected is nucleic acid from EVs, a nucleic acid separation process is further performed. Therefore, the target substance can be efficiently detected using this method with a significantly increased recovery rate of EVs.

### Example 12: Evaluation of Effect of Promoting Scratch-Wound Healing of MDA-MB231 EVs Isolated by Salt Precipitation Method

EVs were isolated from 50 mL of MDA-MB231 culture media for each of methods, SP and UC. For scratch-wound healing assay, MDA-MB231 cells were seeded in 96-well plates at a density of 100,000 cells per well. The cells were grown as a monolayer and a scratch wound was made in the center of the well. Each well was washed several times to remove floating cells, and cells were treated with SP- and UC-isolated EVs at concentrations of 0, 5, 10, and 20 ug/mL (measured by BCA total protein assay) per well. Wounds were imaged with the JuLI Stage Real-time live cell imaging system at 4x magnification at 4-hour intervals for up to 20 hours.

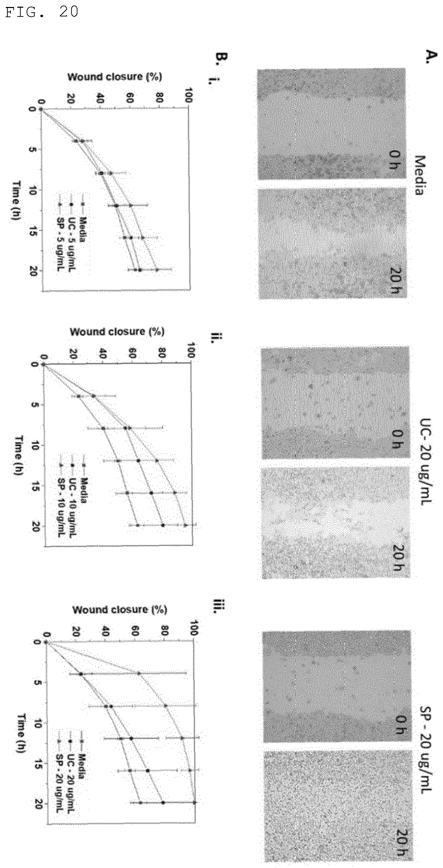

FIG. 20A shows representative images of wound closure when treated with media, EVs isolated by UC, and EVs isolated by SP. The wound closure rate varied depending on the amount of EVs and was higher at a concentration of 20 ug/mL (FIG. 20B). EVs prepared by salt precipitation significantly promoted scratch-wound closure compared to EVs prepared by ultracentrifugation. This suggests that EVs prepared by the salt precipitation method maintain their functional activity.

### Example 13: Comparison of RNA Recovery Rates from EVs Isolated by Ammonium Sulfate and Other Methods

To compare the ammonium sulfate method with other methods for efficient isolation of EVs from biological fluid samples, the ammonium sulfate method was assessed together with other methods such as ultracentrifugation (UC), an exoEasy kit (Qiagen), and a Norgen exosome purification kit. 1 mL of plasma spiked with EV in LN-CM was used for each method. Samples were treated with or without proteinase-K (P-K) prior to EV isolation.

A standard ultracentrifugation process was used for the isolation of EVs by ultracentrifugation (UC). Plasma was centrifuged in a Beckman Coulter Ultracentrifuge at 120,000xg for 90 min at 4°C using a TLA 120.2 Ti fixed-angle rotor (Beckman Coulter) and a 1.2-mL polycarbonate ultracentrifuge tube to pellet EVs. To remove protein contaminants, the supernatant was carefully removed, and the EV pellet was resuspended in PBS and centrifuged again at 120,000xg for 90 min at 4°C. The supernatant was removed and the resulting EV pellet was resuspended in the desired volume of PBS to facilitate further analysis. EV isolation using commercial kits (Qiagen and Norgen) was performed according to the manufacturer's instructions. For EV isolation by AS, single-step precipitation (SP) was used, and the EVs obtained after desalting were used for RNA extraction.

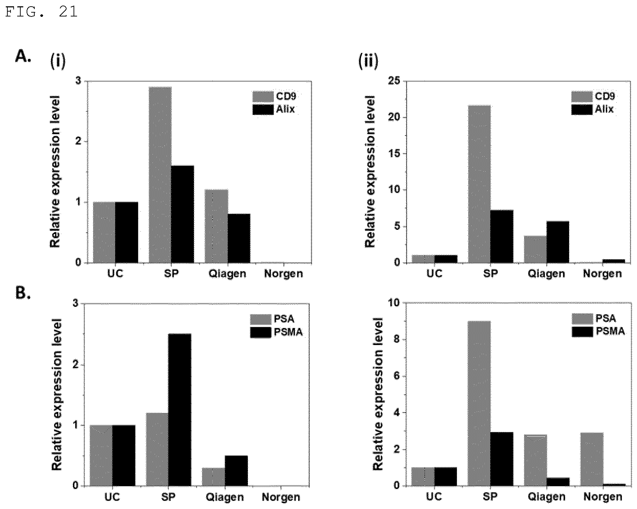

The relative expression levels of EV markers (CD9 and ALIX) and cancer markers (PSA and PSMA) were plotted (FIG. 21). The SP method exhibited the highest expression for all of the markers in both P-K treated and untreated samples when compared to the above test methods.

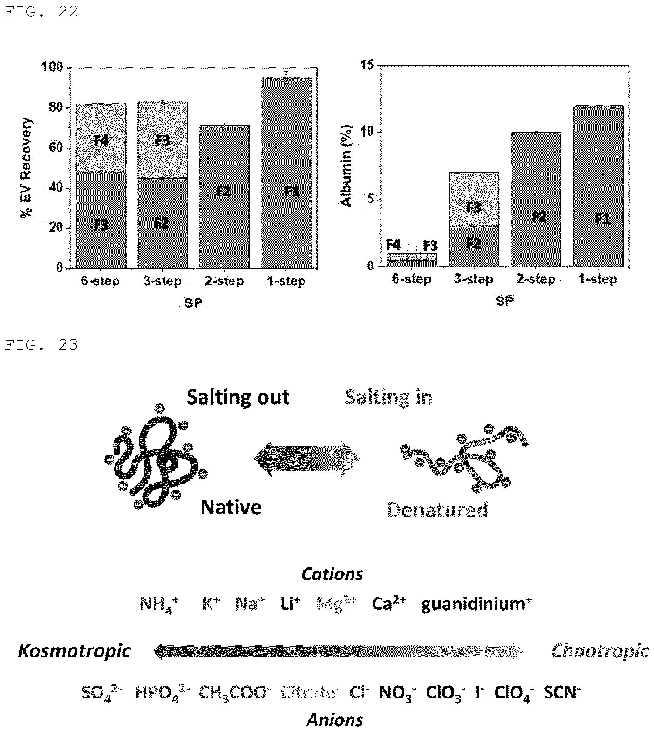

FIG. 22 shows the results of comparing the recovery rate and purity of EVs isolated from plasma using SP methods with different steps shown in the above Examples. As shown therein, increasing the number of steps can improve the purity of isolated EVs while ensuring a similar recovery rate. As shown in the above Examples, SP methods with different steps can be used for EV isolation depending on various sample types and needs such as desired yield and purity, ease of use, etc. For example, as shown in FIGS. 9, 21 and 22, all of the SP methods with different steps ensure a very high recovery rate of more than 80% compared to other EV isolation methods, and as the number of the steps of the SP method is increased, EVs with higher purity can be isolated and enriched.

### Example 13: EV Isolation Using Various Types of Salts

Protein salting-out can occur with various types of neutral salts. Salts interact with water, reducing the number of water molecules available for protein (or EV) solvation. As a result, protein (or EV) interactions increase, resulting in protein (or EV) aggregation and precipitation.

The salting-out abilities of various salts were tested (Table 1). High ion-producing salts from the Hofmeister series were used (FIG. 23). For each salt, either a 4M solution or a saturated solution was prepared, and for salts where the 4M solution was not a saturated solution, both 4M and saturated solutions were prepared (Table 1).

**[Table 1]**

| List of salts used in EV precipitation | | | | | | | |
|---|---|---|---|---|---|---|---|
| | **Salt solution** | **Molecular Formula** | **Mol. wt** | **Solubility at RT (g)/L** | **Wt (g)/25 mL** | **Saturated solution?** | **pH** |
| 1 | Saturated ammonium sulfate | (NH₄)₂SO₄ | 132 | 750 | 18.8 | O | 5.6 |
| 2 | Saturated ammonium phosphate, dibasic | (NH₄)₂HPO₄ | 132 | 600 | 15 | O | 8.0 |
| 3 | 4M Ammonium chloride | NH₄Cl | 53 | 383 | 5.4 | X | 4.3 |
| 4 | Saturated ammonium chloride | | | | 9.6 | O | 4.1 |
| 5 | 4M Ammonium Acetate | CH₃COONH₄ | 77 | 1430 | 7.7 | X | 7.6 |
| 6 | Saturated ammonium Acetate | | | | 35.8 | O | 8.2 |
| 7 | Saturated potassium sulfate | K₂SO₄ | 174 | 120 | 4 | O | 6.0 |
| 8 | 4M Potassium(di) phosphate | K₂HPO₄ | 174 | 1493 | 17.4 | X | 9.1 |
| 9 | Saturated potassium(di) phosphate | | | | 37.4 | O | 9.7 |
| 10 | Saturated potassium chloride | KCl | 75 | 340 | 8.5 | O | 4.6 |
| 11 | 4M Potassium acetate | CH₃COOK | 98 | 2686 | 9.8 | X | 8.8 |
| 12 | Saturated potassium acetate | | | | 67.2 | O | 10.2 |
| 13 | Saturated sodium sulfate | Na₂SO₄ | 142 | 281 | 7 | O | 5.2 |
| 14 | Saturated sodium(di) phosphate | Na₂HPO₄.12H ₂O | 358 | 118 | 3 | O | 8.8 |
| 15 | 4M Sodium chloride | NaCl | 58 | 360 | 5.84 | X | 4.8 |
| 16 | Saturated sodium chloride | | | | 9 | O | 5.8 |
| 17 | 4M Sodium acetate | CH₃COONa | 82 | 464 | 8.20 | X | 9.1 |
| 18 | Saturated sodium acetate | | | | 11.6 | O | 9.0 |
| 19 | Saturated trisodium citrate | Na₃C₆H₅O₇.2 H₂O | 294 | | Sat. solution | O | 8.0 |
| 20 | Saturated magnesium sulfate | MgSO₄.7H₂O | 246 | 1130 | 28.3 | O | 6.2 |

EVs were isolated by incubating equal volumes of cell-conditioned media and salt solution at room temperature for 30 minutes followed by centrifugation at 10,000g for 15 minutes. The pellet resuspended in PBS buffer was then desalted by filtration.

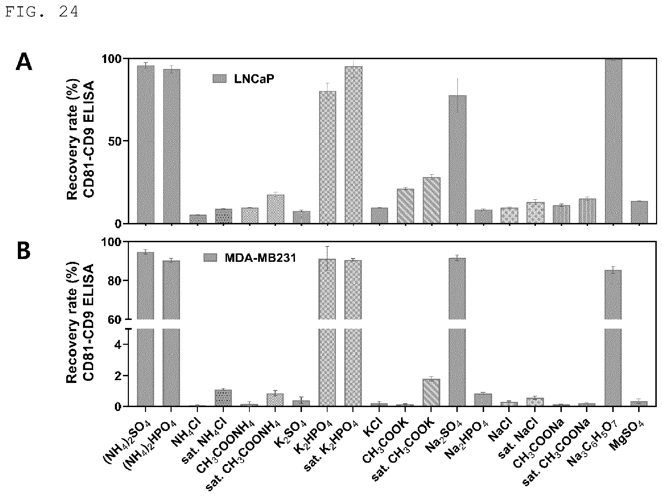

EV recovery rate was calculated using CD81-CD9 sandwich ELISA (FIG. 24). Salts containing a polyvalent anion and a monovalent cation, such as ammonium sulfate, ammonium phosphate, potassium phosphate, sodium sulfate, and trisodium citrate, preferred precipitation and exhibited the highest recovery rate among the salts tested. However, salts such as potassium sulfate and sodium phosphate did not precipitate proteins due to their low solubility in water. In addition, salts with monovalent anions (e.g., chloride and acetate) and salts with polyvalent cations (e.g., magnesium sulfate) did not precipitate any EV even at saturation.

### Industrial Applicability

According to the present invention, it is possible to isolate extracellular vesicles with high purity and high efficiency within a short time in a simpler manner than a conventional extracellular vesicle isolation method.

The extracellular vesicle isolation method according to the present invention has advantages in that it is applicable to various types of biological samples, including samples (plasma, serum, urine, saliva, tissue, cell culture media, ascitic fluid, bronchial lavage fluid, etc.) that can be collected from humans, pigs, mice, rats, etc., and is capable of easily removing salts by washing, unlike a conventional extracellular vesicle isolation method based on precipitation, making it possible to obtain extracellular vesicles with high purity and yield. In addition, the method according to the present invention is applicable to both small-scale isolation of extracellular vesicles for research and diagnostic purposes and large-scale isolation of extracellular vesicles for industrial applications.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

## Claims

1. A method of isolating extracellular vesicles (EVs) from a biological fluid sample, the method comprising:
(a) adding a salt to the biological fluid sample to a concentration ranging from 0.1 M to a saturation concentration;
(b) separating an aggregated or precipitated extracellular vesicle fraction and a supernatant from the sample to which the salt has been added; and
(c) isolating extracellular vesicles by desalting the extracellular vesicle fraction separated in step (b).

2. The method according to claim 1, wherein step (a) is performed by adding the salt 1 to 15 times to a final concentration ranging from 0.1 M to a saturation concentration.

3. The method according to claim 1, wherein step (c) comprises isolating extracellular vesicles from the extracellular vesicle fraction separated from the sample to which the salt has been added to a concentration of 1.4 M to 2.25 M.

4. The method according to claim 1, wherein the salt contains a polyvalent anion and a monovalent cation.

5. The method according to claim 4, wherein the polyvalent anion is sulfate, phosphate, or citrate, and the monovalent cation is an ammonium ion, a potassium ion, or a sodium ion.

6. The method according to claim 4, wherein the salt is ammonium sulfate, ammonium phosphate, potassium phosphate, sodium sulfate, or trisodium citrate.

7. The method according to claim 1, wherein the salt is in a solid or liquid form.

8. The method according to claim 1, wherein step (b) is performed by sedimentation, filtration, or centrifugation.

9. The method according to claim 1, wherein the desalting is performed using filtration, centrifugation, dialysis, reverse osmosis, or a desalting column.

10. The method according to claim 1, wherein an ultrafiltration step, an affinity chromatography step, or a density gradient ultracentrifugation step is further performed on the extracellular vesicle fraction.

11. The method according to claim 1, wherein the biological fluid is selected from the group consisting of serum, plasma, whole blood, urine, saliva, breast milk, tears, sweat, joint fluid, cerebrospinal fluid, semen, vaginal fluid, sputum, pleural fluid, lymph fluid, ascitic fluid, amniotic fluid, bronchial lavage fluid, and media taken from cultured cells.

12. A method of isolating extracellular vesicles (EVs) from a biological tissue sample, the method comprising:
(a) lysing or grinding and clarifying a biological tissue sample;
(b) adding a salt to the clarified sample to a concentration ranging from 0.1 M to a saturation concentration;
(c) separating an aggregated or precipitated extracellular vesicle fraction and a supernatant from the sample to which the salt has been added; and
(d) isolating extracellular vesicles by desalting the extracellular vesicle fraction separated in step (c).

13. The method according to claim 12, wherein step (b) is performed by adding the salt 1 to 15 times to a final concentration ranging from 0.1 M to a saturation concentration.

14. The method according to claim 12, wherein step (d) comprises isolating extracellular vesicles from the extracellular vesicle fraction separated from the sample to which the salt has been added to a concentration of 1.4 M to 2.25 M.

15. The method according to claim 12, wherein the salt contains a polyvalent anion and a monovalent cation.

16. The method according to claim 15, wherein the polyvalent anion is sulfate, phosphate, or citrate, and the monovalent cation is an ammonium ion, a potassium ion, or a sodium ion.

17. The method according to claim 15, wherein the salt is ammonium sulfate, ammonium phosphate, potassium phosphate, sodium sulfate, or trisodium citrate.

18. The method according to claim 12, wherein the salt is in a solid or liquid form.

19. The method according to claim 12, wherein step (c) is performed by sedimentation, filtration, or centrifugation.

20. The method according to claim 12, wherein the desalting is performed using filtration, centrifugation, dialysis, reverse osmosis, or a desalting column.

21. The method according to claim 12, wherein an ultrafiltration step, an affinity chromatography step, or a density gradient ultracentrifugation step is further performed on the extracellular vesicle fraction.

22. The method according to claim 12, wherein the biological tissue is selected from the group consisting of a collection of cells from prokaryotes, eukaryotes, bacteria, fungi, yeast, invertebrates, vertebrates, reptiles, fish, insects, plants or animals, and cultured cells.

23. A kit for isolating extracellular vesicles (EVs) comprising a salt and a buffer.

24. The kit according to claim 23, wherein the salt contains a polyvalent anion and a monovalent cation.

25. The kit according to claim 24, wherein the polyvalent anion is sulfate, phosphate, or citrate, and the monovalent cation is an ammonium ion, a potassium ion, or a sodium ion.

26. The kit according to claim 24, wherein the salt is ammonium sulfate, ammonium phosphate, potassium phosphate, sodium sulfate, or trisodium citrate.

27. The kit according to claim 23, further comprising:
(i) a vessel containing an antibody or ligand that binds to a surface marker exposed on the surface of the extracellular vesicle or to a protein present inside the extracellular vesicle;
(ii) at least one solid support that binds directly or indirectly to a surface marker exposed on the surface of the extracellular vesicle or to a protein present inside the extracellular vesicle; and/or
(iii) a vessel containing at least one salt and at least one buffer for performing density gradient centrifugation of the extracellular vesicles.

28. The kit according to claim 27, wherein the surface marker is selected from the group consisting of HLA DP haplotypes, HLA DQ haplotypes, HLA DR haplotypes, CD9, CD81, CD63, and CD82.

29. The kit according to claim 27, wherein the solid support is resin or beads.

30. The kit according to claim 29, wherein the beads are silica, magnetic particles, polystyrene, or agarose.
